(19) Europäisches Patentamt
European Patent Office
Office européen des brevets

(11) EP 3 929 221 A1

(12) EUROPEAN PATENT APPLICATION
published in accordance with Art. 153(4) EPC

(43) Date of publication:
**29.12.2021 Bulletin 2021/52**

(21) Application number: **20783175.1**

(22) Date of filing: **30.03.2020**

(51) Int Cl.:
***C08F 20/28*** *(2006.01)* ***C12M 1/26*** *(2006.01)*
***C12M 3/00*** *(2006.01)* ***C12N 5/07*** *(2010.01)*
***C12N 5/071*** *(2010.01)* ***C12N 5/0735*** *(2010.01)*
***C12N 5/074*** *(2010.01)* ***C12N 5/077*** *(2010.01)*
***C12N 5/0775*** *(2010.01)* ***C12N 5/0784*** *(2010.01)*
***C12N 5/0786*** *(2010.01)* ***C12N 5/0787*** *(2010.01)*
***C12N 5/0789*** *(2010.01)* ***C12N 5/0793*** *(2010.01)*
***C12N 5/09*** *(2010.01)* ***C12N 1/00*** *(2006.01)*
***C12N 1/02*** *(2006.01)* ***G01N 33/48*** *(2006.01)*
***C12N 11/087*** *(2020.01)*

(86) International application number:
**PCT/JP2020/014538**

(87) International publication number:
**WO 2020/203965 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.03.2019 JP 2019069462**
**18.04.2019 JP 2019079696**

(71) Applicant: **Kyushu University, National University Corporation**
**Nishi-ku**
**Fukuoka-shi**
**Fukuoka 819-0395 (JP)**

(72) Inventors:
- **TANAKA, Masaru**
  **Fukuoka-shi, Fukuoka 819-0395 (JP)**
- **ARATSU, Fumihiro**
  **Fukuoka-shi, Fukuoka 819-0395 (JP)**

(74) Representative: **EP&C**
**P.O. Box 3241**
**2280 GE Rijswijk (NL)**

# (54) PARTICLES FOR CELL ADHESION AND UTILIZATION THEREOF

(57) Particles for cell adhesion to be used for adhering highly adhesive cells present in an aqueous solution, said cell-adhesive particle comprising, in at least a part of the surface thereof, a wettable composition having an intermediate water content in the water-saturated state of 1-30 wt%.

EP 3 929 221 A1

**Description**

TECHNICAL FIELD

The present invention relates to a cell-adhesive particle to be used to selectively adhere and separate predetermined cells present in blood or the like in a living body, and a method for capturing predetermined cells present in blood or the like by selectively adhering the cells using the cell-adhesive particle. The present invention claims priority on the basis of Japanese Patent Application No. 2019-69462, filed in Japan on March 30, 2019, and Japanese Patent Application No. 2019-79696, filed in Japan on April 18, 2019, the contents of which are incorporated herein by reference.

BACKGROUND OF THE INVENTION

Biopsy has been conventionally conducted clinically, in which a tissue is collected from a lesion area to conduct various examinations. In contrast, the tissue is excised in a general biopsy, which is highly invasive to the patient. In contrast, a technique known as "liquid biopsy" has become popular in recent years. In the liquid biopsy, body fluids such as blood or saliva are collected from the patient and analyzed mainly by instrumental analysis relating to target substances contained in the body fluids. Therefore, the liquid biopsy has advantages in that the invasiveness to the patient is reduced and various information can be obtained at a low cost. Although the main examination target was lipids, proteins, or other molecules dissolved in body fluids in the conventional liquid biopsy, tumor cells present in body fluids are required to be collected for the purpose of treating cancer particularly. In addition, it is desirable in the field of regenerative medicine that stem cells present in the body be collected in a less invasive manner as in biopsy to capture and cultivate the stem cells to be used for treatment or the like.

Typical examples of body fluids to be used to collect tumor cells, stem cells, or the like include blood and lymph. In contrast, blood or lymph mainly contains blood cells such as red blood cells, white blood cells and platelets, whilst scarcely containing tumor cells, stem cells, and the like. Therefore, a step in which target cells present in a low amount are selectively collected from blood cells and the like which are present at a high density and in a high amount in body fluid is required to collect the target cells from the body fluid such as blood, and various techniques for conducting such a step have been studied.

Examples of known methods for separating target cells include: a method in which target cells are separated and collected by filtration or centrifugation utilizing the difference in cell size; a method in which target cells are separated and collected using a microfluidic device utilizing the difference in dielectrophoretic properties; and a method in which target cells are collected by making the target cells adhere to the surface on which ligands, such as antibodies against membrane antigens of the target cells, are immobilized, utilizing the selective binding properties of the antibody.

In addition, a method in which target cells are separated by making the target cells adhere to fine particles in which ligands such as antibodies are immobilized has been particularly studied as a method for separating specific cells present at a low density in blood or the like. The method in which fine particles are used as carriers of ligands makes it possible to increase the specific surface area of the surface to which cells are adhered, and therefore is particularly suitable for an application in which cells scarcely present in blood or the like are adhered.

Patent Document 1 discloses, for example, a technique for counting tumor cells circulating in blood (CTC, Circulating Tumor Cells) by selectively adhering and separating CTC using colloidal magnetic particles having a particle size of 90 nm to 150 nm in which anti-EpCAM antibodies against EpCAM (epithelial cell adhesion molecule), which is expressed in many carcinomas, are immobilized (antibody-magnetic particle complexes), and the technique has been put to practical use as Cell Search System.

Patent Document 1 discloses, as characteristics required for a particle that selectively adheres to a target cell such as CTC, that i) an antibody as a biological ligand which reacts specifically with the target cell is adhered on the surface of the particle, ii) the particle can be suspended colloidally in an aqueous solution while having a particle size of 90 nm to 150 nm so as to ensure an opportunity that allows adhesion to a target cell present in a sample, iii) the particle is a magnetic particle so as to allow separation of the target cell adhering to the particle by application of a magnetic field, and iv) the particle is coated with a base coating material in an amount sufficient to prevent a biopolymer present in a living body from adhering to the particle non-specifically (paragraphs 0012 to 0015). In addition, it is disclosed in examples that magnetic particles having a base-coat of bovine serum albumin (BSA) or the like, on which an anti-EpCAM antibody was immobilized, and the particle size of which was 90 nm to 150 nm, preferably functioned (paragraphs 0032 to 0038).

It is further disclosed in Patent Document 1, in which counting of CTC or the like is aimed at, that magnetic particles having the above-mentioned nanometer size sufficiently smaller than the cells do not interrupt the counting, and therefore there is no need to remove the magnetic particles from the cells before analysis (paragraph 0033), and that in the case where particles having a particle size of 28 μm are used, magnetic particles adhering to cells are required to be desorbed by replacing the antibody adhering on the cell surface with a predetermined reagent to desorb only the

magnetic particles, or cutting bonds between the antibodies and the magnetic particles using a predetermined reagent to desorb only the magnetic particles (paragraph 0041).

Thus, the cells obtained by the method disclosed in Patent Document 1 are cells to which nanometer-sized magnetic particles are adhered or cells having a surface structure modified to allow desorption of the magnetic particles, and therefore the use application of the collected cells is limited to mere counting or the like, and is considered to be problematic when used to conduct various biological evaluations after cultivation of collected cells.

Patent Document 2 and the like disclose a technique in which magnetic beads on which a predetermined antibody is immobilized are used as carrier particles to selectively adhere predetermined stem cells. However, it is predicted that a problem similar to that of Patent Document 1 arises, since the stem cells are allowed to adhere to the carrier particles by binding antibodies to the stem cell surface.

In contrast, it has been found by the present inventors that when a wettable composition (hydrated materials) of a group of polymers is hydrated, a part of the hydrated water molecules forms a state defined as intermediate water, and blood components (such as platelets, white blood cells, complements, and clotting factors) have difficulty adhering to the surface on which the intermediate water is present, and thus excellent blood compatibility is exhibited (Non-Patent Document 1). In addition, it has been found that tumor cells such as metastatic cancer cells, stem cells, or vascular endothelial cells contained in blood can adhere to a surface that exhibits difficulty in adhesion of the blood components due to the presence of intermediate water (Patent Document 3). In addition, it has been shown that the surface containing intermediate water at a predetermined rate is suitable to cultivate various cells (Patent Document 4).

## DOCUMENTS OF RELATED ART

### Patent Documents

Patent Document 1: Japanese Translation of PCT Application Publication No. 2002-503814
Patent Document 2: Japanese Patent Application Laid-Open Publication No. 2010-104350
Patent Document 3: Japanese Patent Application Laid-Open Publication No. 2012-105579
Patent Document 4: Japanese Patent Application Laid-Open Publication No. 2016-63801

### Non-Patent Documents

Non-Patent Document 1: Biomaterial 28-1, 2010, p.34-46

## SUMMARY OF THE INVENTION

### PROBLEMS TO BE SOLVED BY THE INVENTION

As described above, in the case where target cells present in the blood or the like are selectively adhered and separated using carrier particles such as magnetic particles, it was an only option to use an antibody against a membrane antigen of the target cells to realize selective adhesion.

On the other hand, an antibody corresponding to target cells should be used in the method in which an antibody against a membrane antigen of target cells is used, and therefore in the case where target cells are not entirely clear or target cells are transformed, the method has problems in that the target cells required to be captured cannot be captured For example, it has been pointed out that the difference in clones of anti-EpCAM antibody used to target CTC causes difference in types of detected CTC. There are problems in that the EpCAM is not always expressed in all CTC and disappears when pre-treatment of the separation treatment or the like is conducted, and therefore the result obtained by the method in which an antibody against a target cell membrane is used may not always reflect the phenomenon of the target.

Furthermore, in the case where target cells adhering to carrier particles via antibodies are desorbed, it is required to conduct a treatment in which a predetermined reagent is replaced with the antibodies or only the carrier particles are desorbed while the antibodies remain on the target cells, which may result in transformation of the target cells, thereby causing interruption on subsequent cultivation or evaluation.

The present invention aims to provide a novel cell-adhesive particle that allows separation of a target cell from blood or the like by selectively adhering a cell such as a tumor cell or stem cell present in the blood or the like without using any antibodies or the like to selectively adhere the target cell. Furthermore, the present invention aims to provide a capture method in which a tumor cell, a stem cell, or the like, which are present in blood or the like, is selectively adhered by using the cell-adhesive particle.

MEANS TO SOLVE THE PROBLEMS

In order to solve the above-mentioned problems, the present invention encompasses the following aspects.

(1) A cell-adhesive particle adherable to highly adherent cells present in an aqueous solution, including a wettable composition having an intermediate water content of 1% by weight to 30% by weight when saturatedly hydrated on at least a part of the surface.
(2) The cell-adhesive particle, wherein the average particle size is 2 μm to 500 μm.
(3) The cell-adhesive particle, wherein the highly adherent cells include tumor cells, stem cells, vascular endothelial cells, nerve cells, macrophages, dendritic cells, monocytes, neutrophils, smooth muscle cells, fibroblasts, cardiac muscle cells, skeletal muscle cells, hepatic parenchymal cells, hepatic nonparenchymal cells, or pancreatic islet cells.
(4) The cell-adhesive particle, wherein the wettable composition is a polymer containing methoxyethyl acrylate.
(5) A cell capture method including adhering highly adherent cells by contacting a particle containing a wettable composition having an intermediate water content of 1% by weight to 30% by weight when saturatedly hydrated on at least a part of the surface with a solution containing the highly adherent cells.
(6) The cell capture method, wherein the average particle size of the cell-adhesive particle is 2 μm to 500 μm.
(7) The cell capture method, wherein the highly adherent cells include tumor cells, stem cells, vascular endothelial cells, nerve cells, macrophage, dendritic cells, monocytes, neutrophils, smooth muscle cells, fibroblasts, cardiac muscle cells, skeletal muscle cells, hepatic parenchymal cells, hepatic nonparenchymal cells, or pancreatic islet cells.
(8) The cell capture method, wherein the wettable composition is a polymer containing methoxyethyl acrylate.
(9) A particle-cell complex, including: a particle containing a wettable composition having an intermediate water content of 1% by weight to 30% by weight when saturatedly hydrated on at least a part of the surface; and highly adherent cells adhered on the surface of the particle.
(10) The particle-cell complex, wherein the average particle size of the particle is 2 μm to 500 μm.
(11) The particle-cell complex, wherein the highly adherent cells include tumor cells, stem cells, vascular endothelial cells, nerve cells, macrophages, dendritic cells, monocytes, neutrophils, smooth muscle cells, fibroblasts, cardiac muscle cells, skeletal muscle cells, hepatic parenchymal cells, hepatic nonparenchymal cells, or pancreatic islet cells.
(12) The particle-cell complex, wherein the wettable composition is a polymer containing methoxyethyl acrylate.
(13) A method for desorbing the cells from the particle-cell complex, including inoculating the particle-cell complex in a cell culture medium.
(14) A cell-cultivation method including cultivating the cells obtained by the above-mentioned method in the cell culture medium.

EFFECTS OF THE INVENTION

The use of the cell-adhesive particle according to the present invention makes it possible to form a complex by adhering a target cell such as a tumor cell or a stem cell in an aqueous solution such as blood, thereby separating and collecting the target cell from the aqueous solution easily. The use of a cell-adhesive particle having a particle size of 2 μm or more causes subsequent self-sustaining desorption of the cell in a culture medium or the like, thereby allowing the target cell to be separated and collected from blood or the like while suppressing damage to the cell, particularly.

BRIEF DESCRIPTION OF DRAWINGS

Fig. 1 is a schematic diagram indicating a structure of an aqueous layer formed on the surface of a wettable composition.
Fig. 2 is a drawing explaining characteristics of each layer constituting an aqueous layer formed on the surface of a wettable composition.
Fig. 3A shows a XPS measurement result of a polystyrene particle before being coated with PMEA.
Fig. 3B shows a XPS measurement result of a polystyrene particle after being coated with PMEA.
Fig. 4A shows a XPS measurement result of a magnetic particle before being coated with PMEA.
Fig. 4B shows a XPS measurement result of a magnetic particle before being coated with PMEA.
Fig. 5 shows changes in adhesion ratio of HT-1080 cells to cell-adhesive particles.
Fig. 6A is an optical microscopic image of HT-1080 cell adhering to cell-adhesive particles.
Fig. 6B is an optical microscopic image of HT-1080 cells adhering to a cell-adhesive particle.
Fig. 7 shows a desorption ratio of HT-1080 cells from cell-adhesive particles.
Fig. 8A is an optical microscopic image of HT-1080 cell adhering to cell-adhesive particles.

Fig. 8B is an optical microscopic image of HT-1080 cells desorbed from cell-adhesive particles.
Fig. 9A is an SEM image of particles after a platelet adhesion test.
Fig. 9B is an SEM image of particles after a platelet adhesion test.
Fig. 9C is an SEM image of particles after a platelet adhesion test.
Fig. 9D is an SEM image of particles after a platelet adhesion test.
Fig. 10 is an optical microscopic image of HT-1080 cells desorbed from cell-adhesive particles.

EMBODIMENTS FOR CARRYING OUT THE INVENTION

(1) Intermediate water

The present invention is characterized by using a wettable composition containing a water molecule in a state referred to as "intermediate water" when hydrated so as to allow selective adhesion to target cells present together with blood cells and the like.

Intermediate water is considered to be an assembly of water molecules in the state in which the freedom thereof is limited by weak interactions with a wettable composition among water molecules of a hydrated wettable composition. It has been confirmed that blood compatibility, such as difficulty in adhesion of blood components such as blood cells, is exhibited on the surface where the intermediate water is present. The mechanism of realization of blood compatibility on the surface on which intermediate water is present is assumed to be as follows.

It is assumed that formation of hydrate shells on the surface of various cells such as blood cells contained in the blood contributes to realization of stabilization, thereby preventing unnecessary activation from being caused by contacting with physiological tissues or the like. In contrast, it is assumed that the hydrate shells cause activation by directly contacting with the surface of a foreign body to be disrupted or destroyed, thereby adhering to the surface of the foreign body. Typical examples thereof include a reaction in which platelets present in a blood vessel stably are activated by contacting with the surface of a foreign body due to bleeding or the like to cause coagulation of the blood.

In contrast, it is assumed that intermediate water forms hydrate shells on the surface of a wettable composition containing the intermediate water, thereby serving as a cushioning that prevents a physiological substance from contacting directly with the surface (Fig. 1), and therefore the degree of disturbance of hydrated shells formed by cells or proteins, which contact with the surface, is decreased to exhibit blood compatibility such as difficulty in adhesion of blood components or the like. Since the presence of intermediate water in the hydrate shells formed on the cell surface or protein has been confirmed, it is considered that the surface of a wettable composition containing intermediate water mimics the surface structure of physiological tissue.

It has been confirmed that the phenomenon in which various cells are prevented from adhering on the surface of the wettable composition containing intermediate water as mentioned above is significantly recognized on the surface of a wettable composition containing a large amount of intermediate water, particularly, and almost all cells present in the blood can be prevented from adhering. It was confirmed in Patent Document 1 that intermediate water is contained at a high ratio in BSA used as a base coating material to prevent non-specific adhesion of biopolymers.

In contrast, it has been observed that adhesion of blood cells such as platelets is suppressed on the surface of the wettable composition having an intermediate water content of approximately 1% by weight to 30% by weight when saturatedly hydrated, whilst particular cells, such as tumor cells, stem cells, or vascular endothelial cells, contained in the blood, adhere thereon. It is assumed that the phenomenon is caused by decreasing hydrate shells formed by intermediate water on the surface in which the intermediate water content is low, thereby allowing only cells having an adherability higher than that of blood cells to significantly adhere for some reason. It is considered that the threshold of the intermediate water content that determines whether or not a cell significantly adheres depends on the adherability of the cell, and it is difficult for ordinary blood cells to adhere on the surface of the wettable composition having an intermediate water content of approximately 1% by weight or more due to the low adherability of ordinary blood cells. The present inventors disclosed in Patent Document 3 a technology in which cells having a high adherability contained in blood, such as tumor cells such as metastatic cancer cells, stem cells, or vascular endothelial cells, are selectively adhered and separated from blood cells or the like utilizing the phenomenon.

The cells that can adhere even on the surface of the wettable composition having an intermediate water content of approximately 1% by weight or more, such as tumor cells, stem cells, or vascular endothelial cells, may be described as "highly adherent cells" in the present specification. In addition, it may be described as simply "selectively adhere" or the like to adhere the highly adherent cells without substantially adhering blood cells or the like present at a high density in the blood.

It is predicted that tumor cells or the like can be adhered and separated without damaging the cells by the method disclosed in Patent Document 3, in comparison with the method in which particular cells are adhered using antibodies disclosed in Patent Document 1 or 2, because the surface containing intermediate water at a predetermined ratio is suitable as a culture substrate for various cells, as disclosed in Patent Document 4.

Thus, the use of the wettable composition having an intermediate water content of approximately 1 % by weight to 30% by weight when saturatedly hydrated in a flat membrane makes it possible to selectively adhere highly adherent cells such as tumor cells, stem cells, or vascular endothelial cells and separate the highly adherent cells from blood cells or the like. It is further predicted that highly adherent cells can be adhered and separated efficiently as targets due to an increase in specific surface area by applying the finding on the flat membrane of the wettable composition to adhesion and separation of target cells using carrier particles.

(2) Cell-adhesive particle according to the present invention

However, in the case of an attempt in which cells scarcely present in blood or the like are made to adhere to carrier particles having adherability to various cells, as studied particularly in Patent Document 1 or the like, a predetermined stirring process or the like is required to ensure collision frequency between the carrier particles and the cells, and it is not clear whether or not various target cells adhere to a wettable composition containing intermediate water and then the adhesion state is maintained in a dynamic environment in which the stirring process is conducted. In other words, although it was disclosed in Patent Document 3 that the adhesion between the wettable composition and various cells was realized in a quasi-static environment, it was not clear whether or not sufficient adhesion was realized in a dynamic environment.

In contrast, it has become clear by the present inventors that when particles having a predetermined intermediate water content on the surface thereof are added to and mixed with an aqueous solution in which highly adherent cells are present, the cells and the particles are adhered at an unexpectedly sufficient frequency to form complexes, and the complexes can be separated by centrifugation or the like from the aqueous solution. In addition, it has been shown that no platelets adhere to the surface of the particles containing intermediate water in a similar manner to that of the flat membrane, and therefore highly adherent cells present together with blood cells in an aqueous solution such as blood can be selectively adhered and separated using particles having a predetermined intermediate water content on the surface thereof, in a similar manner to that where the flat membrane of the wettable composition is used, thus realizing the present invention.

From the above-mentioned finding, an invention is specified in which a specific cell present in an aqueous solution, such as blood, can be separated from the aqueous solution by making the cell selectively adhere to a particle, in which at least a part of the surface is composed of a wettable composition having an intermediate water content of 1 % by weight to 30% by weight when saturatedly hydrated, to form a complex, followed by conducting centrifugation or the like. In addition, an invention is specified in which a particle in which at least a part of the surface is composed of a wettable composition having an intermediate water content of 1% by weight to 30% by weight when saturatedly hydrated is used to adhere cells.

In addition, it has been observed that when complexes composed of the cell-adhesive particles and cells are put into cell culture medium, the cells are allowed to desorb from the particles self-sustainingly and to move upward in the culture medium by adjusting the particle size of the particles to an appropriate range. In other words, it has been found that in the case where cell-adhesive particles having a particle size of 2 or 3 μm or more, which is approximately at least the same size as that of the cells, are used to form complexes composed of the particles and cells, and the complexes are put in cell culture medium and maintained therein for a predetermined time, a certain ratio of the cells desorbs from the particles self-sustainingly to move into the culture medium, which allows subsequent ordinary cell cultivation without conducting any treatments that affect the cell surface. It has been observed particularly that in the case where the particle size of cell-adhesive particles is at least 10 μm, almost all cells desorb self-sustainingly from the particles approximately 2 hours after putting in culture medium, which allows various highly adherent cells present in the blood or the like to be collected efficiently to be used to conduct biological investigation.

It is preferable that particles having a relatively spherical shape having little surface irregularity be used in cell-adhesive particles according to the present invention from the viewpoint that the desorption of cells adhering to the particles is not inhibited. The particle size according to the present invention is an average of values of particle size measured by DLS (Dynamic Light Scattering) (or SLS (Static Light Scattering)).

In other words, it is preferable that the average particle size of the cell-adhesive particles according to the present invention be at least 2 μm to 3 μm from the viewpoint that cells adhered to the cell-adhesive particles and then separated are allowed to desorb self-sustainingly in cell culture medium. It is particularly preferable that the average particle size be at least 5 μm, or at least 10 μm, from the viewpoint that a large ratio of the cells is allowed to desorb self-sustainingly from the particles within a short period of time.

In contrast, it is preferable that the particle size not be larger than a size that causes precipitation for a short time depending on the density of the used particles, since the cell-adhesive particles according to the present invention are used by dispersing (suspending) in an aqueous solution such as blood. For example, in the case where a cell-adhesive particle is mainly composed of a polymer such as polystyrene, the particle size of approximately 500 μm or less makes it possible to realize dispersion in an aqueous solution by gentle agitation. In contrast, in the case where the

center particle of the cell-adhesive particle is composed of a high-density substance such as a magnetic particle, the particle size of approximately 50 μm or less makes it possible to realize dispersion in an aqueous solution by gentle agitation.

It is generally preferable that the particle size of the cell-adhesive particle according to the present invention be large within the above-mentioned range, from the viewpoint that complexes obtained by adhesion of various cells to the cell-adhesive particles are to be separated from an aqueous solution by centrifugation or filtering. In contrast, in the case where the center particle of the cell-adhesive particle is composed of a high-density substance, separation can be conducted by centrifugation or the like even when the particle size is fine. In the case where a magnetic particle is made to be the center particle, separation can be conducted favorably utilizing a magnetic field regardless of the particle size.

Although it is preferable that the particle size variation of the cell-adhesive particles to be used be small, the presence of particles having a particle size of 2 μm or less particularly decreases the efficiency of self-sustaining desorption of cells adhering to the particles, and therefore monodispersible particles having an average particle size of at least 2 μm, the particle size distribution of which is narrow, are preferable.

(3) Separation of cells from an aqueous solution using cell-adhesive particles according to the present invention

Complexes obtained by adhesion of various cells to the cell-adhesive particles according to the present invention in an aqueous solution can be separated from the aqueous solution by various methods. For example, the cell-adhesive particles of the complexes can be enriched and separated from the aqueous solution by centrifugation. In the case where magnetic particles are contained in the cell-adhesive particles, separation can be conducted utilizing a magnetic field. In the case where cell-adhesive particles having a predetermined particle size or more are used, separation from an aqueous solution can be conducted by filtration using a filter.

The cells adhering to the cell-adhesive particles separated form an aqueous solution may be used, depending on the purpose, directly while adhering to the cell-adhesive particles, or after the cells are desorbed from the cell-adhesive particles. For example, a wettable composition containing intermediate water at a predetermined ratio exhibits excellent characteristics as a cell culture substrate, as shown in Japanese Patent Application Publication No. 2016-63801 mentioned above, and therefore cells can be cultivated in culture medium while adhering to the cell-adhesive particles according to the present invention. In contrast, in the case where the cell-adhesive particles, the average particle size of which is at least 2 μm, are used, the cells desorb self-sustainingly in culture medium, as mentioned above, and therefore ordinary cell cultivation can be conducted by putting complexes composed of the cell-adhesive particles and the cells in culture medium.

In the case where a wettable composition having a lower critical solution temperature (LCST) at a predetermined temperature region is used as a wettable composition containing intermediate water, as mentioned in Japanese Patent Application Publication No. 2016-131561, for example, separated target cells can be desorbed from cell-adhesive particles by separating target cells from an aqueous solution and then conducting cooling to a predetermined temperature to dissolve the wettable composition. In addition, cells can be desorbed from cell-adhesive particles by conducting washing, agitation, treatment using a chelating agent such as EDTA (ethylenediaminetetraacetic acid) or EGTA (ethylene glycol tetraacetic acid), ultrasonic treatment or the like.

Thus, the use of the cell-adhesive particles according to the present invention makes it possible to form complexes by adhesion of target cells such as tumor cells or stem cells, present at a low density in an aqueous solution such as blood, thereby making it possible to separate and collect easily the cells from the aqueous solution. It is possible according to the present invention to separate and collect target cells without causing change due to the use of an antibody in comparison with the method in which an anti-EpCAM antibody or the like is used. In addition, the use of cell-adhesive particles having a particle size of at least 2 μm causes self-sustaining desorption of cells in culture medium, thereby making it possible to separate and collect the target cells from the blood or the like while suppressing damage to the cells, particularly.

Hereinafter, each aspect of the present invention will be described further specifically.

(4) Measurement method of intermediate water content

The present invention is characterized in that a wettable composition containing 1 % by weight to 30% by weight of intermediate water when saturatedly hydrated by bringing the wettable composition into contact with an aqueous solution such as blood, lymph fluid, or saline, is used. It is apparent that the state of hydrated water molecule when the wettable composition is saturatedly hydrated is broadly classified into three types (Fig. 2).

Water molecules that are the most strongly affected by the wettable composition are in the state that may be referred to as "crystallized water" in which the water molecules are strongly bound by molecules in the composition and in the state in which the water molecules are not allowed to move freely, and cannot freeze to form solid-phase water (ice) even when cooled to extremely low temperature, and therefore are classified as "non-freezing water". All hydrated

water molecules generally form non-freezing water in an initial hydration stage (in which the hydration amount is low).

In contrast, in the saturatedly hydrated wettable composition there are water molecules that are scarcely bound by molecules in the composition and behave in a similar manner to that of water molecules in pure water, such water molecules being classified as "free water". The free water is characterized by causing coagulation and dissolution at around 0°C in a similar manner to pure water.

On the other hand, it has been shown that there are water molecules that exhibit behavior that is not classified as the above-mentioned "non-freezing water" or "free water" when a wettable composition containing a biologically relevant substance such as protein or a specific synthetic polymer is hydrated, such water molecules being classified as "intermediate water".

The intermediate water is typically characterized by the specific release or absorption of latent heat that is generated in a temperature rising process after supercooling. In other words, the specific release or absorption of latent heat, such as the release of latent heat at a temperature of around -50°C to -20°C or the absorption of latent heat at a temperature of -15°C to 0°C, is observed in a process in which a substance containing intermediate water is cooled or heated in a temperature region of - 100°C to -20°C. Such a release or absorption of latent heat can be observed quantitatively using a DSC (differential scanning calorimeter) or the like.

The release of latent heat at a temperature of around -50°C to -20°C is caused to respond to latent heat generated by transformation from an irregular state to a regular state (CC: cold crystallization) when intermediate water coagulated in the irregular state by quenching is gradually heated. The absorption of latent heat at a temperature region of -15°C to 0°C is caused to respond to latent heat generated by phase transformation (melting) of intermediate water in the regular state and melting of free water.

The intermediate water content in the wettable composition may be calculated from the amount of latent heat transfer (enthalpy change) caused by the phase transformation of intermediate water. Specifically, the amount of latent heat release (ΔHcc) measured particularly at around -50°C to -20°C in a process in which the amount of latent heat transfer during cooling or heating a hydrated wettable composition at a temperature range of -100°C to -20°C is measured using a DSC or the like is divided by latent heat of melting water (Cp: 334 J/g) in accordance with formula (1) to obtain the intermediate water content (Wfb) in the wettable composition.

$$\text{Wfb (g)} = \Delta\text{Hcc (J)} / \text{Cp (J/g)} \qquad (1)$$

In the case where the amount of free water (Wf) contained in the wettable composition can be calculated from the amount of latent heat release at around 0°C in the cooling process, for example, the intermediate water content in the wettable composition can be calculated by subtracting the amount of latent heat absorption predicted to be caused by free water from the amount of latent heat absorption (ΔHm) at a temperature region of -15°C to 0°C in the above-mentioned heating process to obtain the amount of latent heat absorption caused by melting intermediate water in the regular state, and then dividing the obtained amount by the value of latent heat of melting water.

Since the phase transformation is not generated in the non-freezing water contained in the wettable composition at a temperature range in which measurement using a DSC or the like is conducted, it is difficult to calculate the amount of non-freezing water (Wnf) from the amount of latent heat transfer. Thus, the amount of non-freezing water contained in the wettable composition is calculated by subtracting the value corresponding to the amounts of intermediate water and free water from the total hydration amount (Wc).

Since the cell-adhesive particle according to the present invention is used by being brought into contact with an aqueous solution such as blood, the wettable composition on the surface of the cell-adhesive particle is saturatedly hydrated when used, and the intermediate water content at the time is determined as the saturated intermediate water content (SWfb).

Some of the results obtained by measuring the saturated intermediate water content in various wettable compositions are shown in Table 1. Since the saturated intermediate water content varies depending on the molecular weight of a polymer used for measurement or the like, and does not correspond to a constant physical property value, it is desirable to confirm every intermediate water content in the wettable composition to be used.

Table 1

| Wettable composition | Saturated intermediate water content (wt%) |
|---|---|
| PMEA | 4.0 |
| PMC3A | 3.5 |
| PHEMA | 12.0 |

(continued)

| Wettable composition | Saturated intermediate water content (wt%) |
|---|---|
| PMPC | 62.0 |
| BSA | 78.0 |
| PMEA: Poly(2-methoxyethylacrylate)<br>PHEMA: Poly(2-hydroyethylmethacrylate)<br>PMC3A: Poly(3-methoxypropylacrylate)<br>PMPC: Poly(2-methacryloyloxyethylphosphorylcholine)<br>BSA: Bovine serum albumin | |

The intermediate water is contained in various wettable compositions, as shown in Table 1, and it is known that the saturated intermediate water content varies depending on the molecular structure. In addition, it is known that intermediate water is present at a high ratio in a polymer derived from a biological body, such as BSA.

Since the above-mentioned PMEA, PMC3A, PHEMA and the like are non-water soluble, the use thereof on the surface of the cell-adhesive particle makes it possible to adhere the highly adherent cells. It is known that PMPC contains intermediate water at a high ratio, while a simple body thereof is water-soluble. Thus, synthesis of a copolymer by mixing non-water soluble monomers makes it possible to provide non-water solubility and to appropriately decrease the saturated intermediate water content, and therefore is suitable to prepare cell-adhesive particles by adjusting the intermediate water content.

(5) Structure of cell-adhesive particle according to the present invention

In the cell-adhesive particle according to the present invention, the presence of a wettable composition having a saturated intermediate water content of 1% by weight to 30% by weight on at least a part of the particle surface allows selective adhesion of target cells, and the internal structure thereof is not particularly limited. In other words, the cell-adhesive particle according to the present invention may adopt an appropriate structure, such as a particle obtained by granulating a wettable composition having a saturated intermediate water content of 1% by weight to 30% by weight into a predetermined particle size by an appropriate procedure, a particle obtained by coating a center particle formed of an appropriate material with a wettable composition having a saturated intermediate water content of 1% by weight to 30% by weight, or a particle obtained by granulating a mixture composed of a wettable composition having a saturated intermediate water content of 1% by weight to 30% by weight and fine particles formed of an appropriate material into a predetermined particle size by an appropriate procedure.

The structure formed by coating a center particle formed of an appropriate material with a wettable composition having a saturated intermediate water content of 1% by weight to 30% by weight makes it possible to form a cell-adhesive particle, almost the entire surface of which is formed by the wettable composition, while various characteristics can be provided depending on the material of the center particle. For example, coating of a magnetic particle as a center particle with the wettable composition makes it possible to conduct separation from an aqueous solution by enriching a cell-adhesive particle to which a predetermined target cell adheres by applying a magnetic field thereto. In addition, it is effective to coat center particles composed of a material having an appropriate unit mass with the wettable composition when cell-adhesive particles adhering predetermined target cells are separated from an aqueous solution or the like by centrifugation.

Coating of center particles with the wettable composition may be conducted by an appropriate procedure, and is not limited particularly. For example, a procedure in which center particles are put into a solution in which the wettable composition is dissolved in a solvent and then stirred, followed by separating and drying the particles may be adopted. In the case where a polymer is used as the wettable composition, center particles may be coated with a predetermined wettable composition by causing polymerization using a polymerization initiator or the like while placing center particles in a solution in which monomers that can produce the polymer by polymerization are dissolved. In addition, it is preferable that a pre-treatment in which the affinity with the wettable composition is improved be conducted on the surface of the center particles.

A wettable composition having a saturated intermediate water content appropriate to the purpose may be used in the cell-adhesive particle according to the present invention. For example, an organic polymer having a predetermined intermediate water, an inorganic material such as hydroxyapatite, a protein such as gelatin, collagen, or albumin, or a polysaccharide such as hyaluronic acid or chondroitin sulfate may be used. In addition, polyethylene glycol (PEG), polyvinylpyrrolidone (PVP), polymethylvinyl ether (PMVE), poly(2-methacryloyloxyethyl phosphorylcholine), poly(tetrahydrofurfuryl acrylate), or poly(oxazoline), which are known as biocompatible polymers, may be used in a condition

in which effects on the human body are suppressed. Preferable examples of the polymers include poly(2-ethoxyethyl acrylate), poly(2-methoxyethyl acrylate), poly[2-(2-methoxyethoxy)ethyl methacrylate], poly[2-(2-ethoxyethoxy)ethyl acrylate], poly[2-(2-methoxyethoxy)ethoxy]ethyl methacrylate, poly[2-(2-(2-methoxyethoxy)ethoxy)ethyl acrylate], and poly[2-(2-ethoxyethoxy)ethyl methacrylate], which are represented by the following formula (1), polyethylene glycol (PEG), polyvinylpyrrolidone (PVP), polymethylvinyl ether (PMVE), methoxyethyl (meth)acrylamide, methoxyethyl vinyl ether, poly(tetrahydrofuryl (meth)acrylate), and poly(oxazoline).

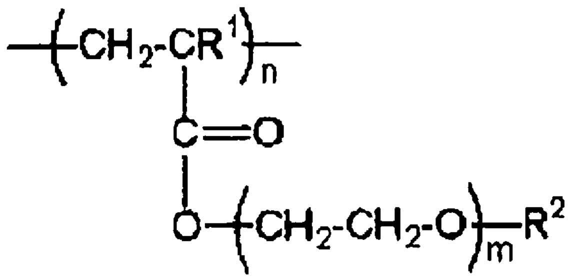

(1)

(In the formula, $R^1$ is a hydrogen atom or a methyl group, $R^2$ is a methyl group or an ethyl group, m1 is 1 to 6, and n is a repeating unit.)

Among these polymers, poly(2-methoxyethyl acrylate) (PMEA), which is represented by the following formula (2)), or the like is particularly preferable in that the biocompatibility thereof is excellent.

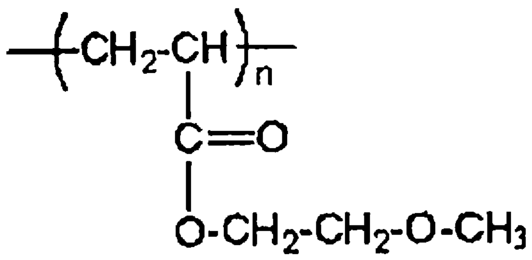

(2)

In addition, monomers constituting these polymers may be mixed with other monomers to form a copolymer, which may be used as a material having a predetermined intermediate water content.

For example, poly [2-(2-ethoxyethoxy)ethyl acrylate] (PEEA) has both an intermediate water and a lower critical solution temperature (LCST) of 14°C, and is characterized by dissolutionability in an aqueous solution at a temperature no higher than the LCST. In the case where a wettable composition has both a predetermined saturated intermediate water content and a lower critical solution temperature (LCST), the wettable composition varies from hydrophobicity to hydrophilicity depending on the temperature, and makes it possible to easily desorb cells by cooling cell-adhesive particles, to which the cells adhere, in an aqueous solution when the wettable composition is provided on the surface of the cell-adhesive particles.

Highly adherent cells present in body fluid such as blood are adhered by the cell-adhesive particle according to the present invention. Examples of the highly adherent cells include tumor cells such as metastatic cancer cells present in blood and leukemia cells. In addition, stem cells, vascular endothelial cells, nerve cells, macrophages, dendritic cells, monocytes, neutrophils, smooth muscle cells, fibroblasts, cardiac muscle cells, skeletal muscle cells, hepatic parenchymal cells, hepatic nonparenchymal cells, or pancreatic islet cells may be detected.

As mentioned above, it is determined whether or not each cell adheres to the wettable composition constituting the particle surface depending on the saturated intermediate water content of the wettable composition, and an increase in the saturated intermediate water content of the wettable composition contributes to suppression of the adhesion of cells having a lower adherability. Thus, it is preferable in terms of improvement of selectivity to increase the saturated intermediate water content of the wettable composition to be used in the cell-adhesive particle according to the present invention within a range in which adhesion of target cells is allowed. In contrast, the use of a wettable composition having a relatively low saturated intermediate water content of approximately 3% by weight suppresses adhesion of the majority of cells suspending on the blood, while makes it possible to adhere and enrich cells scarcely present in the blood. Thus, it is preferable that the saturated intermediate water content on the surface of the cell-adhesive particle be determined depending on the purpose of separating target cells by the cell-adhesive particle according to the present invention or the type of target cells to be separated and the wettable composition having the saturated intermediate water content be selected to be used.

(6) Adhesion of cells using the cell-adhesive particles according to the present invention

The cell-adhesive particles according to the present invention are used by adding the cell-adhesive particles at a predetermined ratio to a body fluid, such as blood or lymph, in which the presence of target cells to be separated is predicted, as a sample. An appropriate pretreatment, such as preliminary dilution, enrichment by centrifugation, or addition of anticoagulants may be conducted on the blood or the like, to which the cell-adhesive particles are added, so as to facilitate the adhesion process between the cell-adhesive particles and the target cells.

It is desirable to conduct agitation under appropriate conditions so as to increase the frequency of collisions between cell-adhesive particles and target cells after the cell-adhesive particles are added at a predetermined ratio to a sample. It is preferable that the agitation conditions allow used cell-adhesive particles to disperse in the sample without precipitating, but the agitation at an excessive rate is not preferable from the viewpoint that an increase in the relative rate of cell-adhesive particles to target cells inhibits adhesion of the cells and the desorption of target cells from cell-adhesive particles is caused.

After agitation is conducted in a sample for a predetermined period of time, cell-adhesive particles adhering target cells are separated from a sample by an appropriate method. The method for separating cell-adhesive particles may be appropriately determined depending on the size, density or physical properties of the cell-adhesive particles. For example, cell-adhesive particles may be deposited by centrifugation, and may be collected by filtration through a filter. In addition, cell-adhesive particles can be collected using cell-adhesive particles containing a magnetic material by applying a magnetic field thereto.

It is preferable that collected cell-adhesive particles, to which target cells adhere, be put into PBS to protect the cells. In the case where the cell-adhesive particles having a particle size of 3 μm or more are particularly used, target cells can be obtained without being damaged by putting particles, to which target cells adhere, into culture medium for cell-adhesion to cause self-sustaining desorption of the cells. In addition, a treatment to be conducted to desorb cells from a substrate, such as treatment using a chelating agent such as EDTA or EGTA, ultrasonic treatment, shaking, or washing, may be conducted to desorb cells from cell-adhesive particles to be subjected to various evaluations.

The cells desorbed from the cell-adhesive particles contain various types of cells present in blood or the like, and therefore target cells are preferably separated therefrom under a microscope to be used. Among the separated cells, tumor cells or the like may be particularly used as indicators to determine the extent and characteristics of tumor progression or possibility of metastases. In addition, the tumor cells desorbed from the cell-adhesive particles may be used to identify the primary tumor side and to screen candidate substances having medicinal potency as anticancer agents by cultivating the tumor cells to proliferate the tumor cells. In addition, stem cells or the like may be used in regenerative medicine by cultivating the stem cells in a predetermined environment.

The cell-adhesive particles according to the present invention may be used to separate target cells by filling a column with the cell-adhesive particles. Specifically, target cells may be separated from a sample such as body fluid or the like by conducting a step of adhering target cells to cell-adhesive particles in a column, and a step of eluting the target cells from the cell-adhesive particles in the column, for example.

The column size, conditions for adhering target cells to cell-adhesive particles in a column, conditions for eluting the target cells from the cell-adhesive particles in a column, or the like, may be determined appropriately.

For example, body fluid or the like, containing target cells, may be brought into contact with cell-adhesive particles filling a column in the step of adhering target cells to cell-adhesive particles in a column, as mentioned above. The body fluid or the like may be preliminarily subjected to dilution, centrifugation, an appropriate pretreatment, or the like.

For example, culture medium may be used as an eluate, an eluate containing a chelating agent may be used, or an appropriate procedure may be adopted in the step of eluting target cells from cell-adhesive particles in a column, as mentioned above.

## Examples

The present invention will be described specifically with reference to examples below. The present invention is not limited to the following examples.

### 1. Synthesis of wettable composition and measurement of intermediate water content

A poly(2-methoxyethyl acrylate) (PMEA) material known to contain intermediate water when hydrated was synthesized.

Polymerization of 15 g of 2-methoxyethyl acrylate was allowed to proceed at 75°C for 10 hours in 60 g of 1,4-dioxane using azobisisobutyronitrile (0.1% by weight) as an initiator while conducting nitrogen bubbling. After the polymerization reaction ended, the resultant was added dropwise into n-hexane to cause precipitation, and the precipitate was isolated. The isolated product was dissolved in tetrahydrofuran, and then subjected to purification twice using n-hexane. The purified product was dried under reduced pressure during the entire day. A colorless and transparent starch syrup-like polymer was obtained. The yield amount (yield ratio) was 12.3 g (82%). The resultant polymer structure was confirmed by 1H-NMR. It was confirmed by the molecular weight analysis by GPC that the number-average molecular weight (Mn) was 26,000 and the molecular weight distribution (Mw/Mn) was 3.27. The PMEA was soluble in methanol and insoluble in water.

The synthesized PMEA was immersed in water for up to 7 days to become sufficiently hydrated, and then the saturated intermediate water content in the PMEA was measured by the following method. The measurement was conducted by taking a predetermined amount of the hydrated wettable composition and spreading thinly on the bottom of a preweighed aluminum oxide pan, followed by measuring the endothermic calorific value caused by the wettable composition sample while changing the temperature in a differential scanning calorimeter (DSC). In the measurement in DSC, the endothermic calorific value was measured as a function of temperature in a process in which the wettable composition sample was cooled from room temperature to -100°C at a cooling rate of 5°C/min and then maintained for 10 minutes, followed by heating the wettable composition sample from -100°C to 50°C at a temperature-rising rate of 5°C/min. The total content (g) of intermediate water contained in the wettable composition sample was determined from the amount of latent heat release (ΔHcc) at around -50°C to -20°C in accordance with the above-mentioned formula (1).

Then, the weight (W1: g) of the hydrated wettable composition sample after DSC measurement was measured, followed by forming a pinhole in the aluminum oxide pan, drying sufficiently the resultant in a vacuum, and then determining the dry weight (Wo: g) to determine the weight decrease as the hydration amount (Wc: g) of the wettable composition sample. The intermediate water content (wt%) in the wettable composition sample was calculated by dividing the total amount (g) of the intermediate water by the dry weight (Wo: g) of the wettable composition sample.

The above-mentioned measurements were performed multiple times so as to avoid various measurement errors, and the maximum value of the obtained intermediate water content was used as the saturated intermediate water content (SWfb) in PMEA. The saturated intermediate water content of PMEA used in the following evaluation was 4.0 wt%.

2. Preparation of cell-adhesive particles

Magnetic particles and polystyrene particles having various particle sizes were used as center particles to prepare cell-adhesive particles by coating the surface of the center particles with the synthesized polymer (PMEA) by the following procedure. Particles (model number: DB6550, DB14203, DB14011) manufactured by Thermo Fisher Scientific, Inc., were used as the magnetic particles (particle size: 1.0 μm, 2.8 μm, 4.5 μm). Particles (model number: 18329-5, 07315-5, 19825-1) manufactured by Poly sciences Inc., were used as the polystyrene particles (particle size: 20 μm, 90 μm, 200 μm to 300 μm). It was confirmed that intermediate water was absent in both the used polystyrene particles and the used magnetic particles.

Each of the particle types dispersing in the aqueous solution was subjected to centrifugation to remove the supernatant and then washing by adding methanol thereto three times to replace the dispersion medium with methanol. Then, each of the particle types after removing the supernatant by centrifugation was put into a methanol solution of PMEA (PMEA concentration: 0.2% by weight), respectively, and then placed in a rotator to conduct gentle agitation for 1 day. Then, a procedure in which the supernatant was removed by centrifugation and then distilled water was added thereto was repeated three times to replace the dispersion medium with distilled water. In addition, a procedure in which the supernatant was removed by centrifugation and then phosphate-buffered saline (PBS) was added thereto was repeated three times to replace the dispersion medium with PBS, followed by storing the resultant at 4°C. Each of the particle types was monodispersed in the resultant dispersion liquid.

Results of XPS (X-ray Photoelectron Spectroscopy) measurement of polystyrene particles (20 μm) and magnetic particles (4.5 μm) before and after PMEA coating are shown in Figs. 3 and 4. The XPS measurement was conducted by using: samples prepared by applying a dispersion liquid in which particles coated with PMEA were dispersed in PBS on glass plates, followed by drying the resultant in a thermostatic bath (37°C) for approximately 10 hours; and AlKa ray (1486.7 eV) as an X-ray source. In addition, XPS measurement was conducted for comparison in a similar manner except that samples prepared by applying an aqueous solution in which each of the particle types uncoated with PMEA were redispersed in PBS on glass plates and then drying the resultant were used.

It was confirmed as shown in Figs 3 and 4 that both the polystyrene particles and the magnetic particles caused clear changes in the results of XPS measurement between before and after conducting PMEA coating treatment, and that approximately the same measurement results were obtained by the polystyrene particles and the magnetic particles after conducting PMEA coating treatment. It was identified that the peak at around 535 eV, significantly observed on the polystyrene particles and the magnetic particles, after conducting the PMEA coating treatment, was derived from oxygen atoms (O1s), and the peak at around 287 eV was derived from carbon atoms (C1s), and therefore it was assumed that the surface of both particles was coated with PMEA by the PMEA coating treatment.

3. Evaluation of adhesion of cancer cells to each particle type and effects on the cells by adhesion

The cell-adherability possessed by the cell-adhesive particles according to the present invention and the state of cells after adhering to the particles and then being separated therefrom were evaluated by using human fibrosarcoma cells (HT-1080) present in PBS so as to evaluate their potential to adhere and separate CTC present in blood. The human fibrosarcoma cells used to conduct evaluation were prepared by being inoculated in a culture medium containing 10% fetal bovine serum (FBS), cultivated in an incubator at 37°C in a 5% carbon dioxide atmosphere, released using 0.25% EDTA/ trypsin every 3 days, reinoculated such that the cell count became 1/8, and then subcultured until approximately 80% confluence was obtained.

(1) Cell adhesion test of each particle type

After the culture medium was removed from the cultivated HT-1080 cells, and then the cells were washed with an appropriate amount of PBS, an appropriate amount of 0.25% EDTA/trypsin was added thereto and then the cells were left undisturbed at 37°C in a 5% carbon dioxide atmosphere for 1 minute to 2 minutes. Then, an appropriate amount of culture medium containing 10% FBS was added to the cells, and then the cells were suspended, collected in centrifuge tubes, and then subjected to centrifugation at room temperature at 1,000 rpm for 3 minutes to remove the supernatant, followed by adding PBS thereto to resuspend the cells. The cells were washed by conducting centrifugation to remove the supernatant and then adding PBS thereto again to obtain a PBS suspension containing HT-1080 cells (the cell concentration in the suspension: $2\times10^6$ cells/ml). The cell concentration was measured using a T20 full automatic cell counter (BIORAD, 1450101J1).

Each of the particle types and HT-1080 cells were adhered by mixing the PBS suspension containing each of the particles coated with PMEA as described above with the PBS suspension containing HT-1080 cells and then placing the mixture on a rotator to conduct agitation.

In the cell adhesion test, each suspension in which each of the particle types was dispersed, the number of the particles being shown in Table 2, was mixed with $5\times10^5$ HT-1080 cells (0.25 ml as each suspension), and the mixture was put in each tube, fixed in a rotator, and then rotated for a predetermined period of time at room temperature at 3 rpm to allow the HT-1080 cells to adhere to the particles. The number of each of the particles was adjusted such that the total surface area became approximately identical to each other. After a predetermined time passed, cell-adhesive particles were precipitated and separated by centrifugation to measure the number (concentration) of cancer cells contained in the supernatant, and the adhesion ratio of the cancer cells to each of the particles was calculated by comparing with the number of added cancer cells.

Table 2

| Particle size (μm) | 4.5 (DN) | 20 (PS) | 90 (PS) | 200 to 300 (PS) |
|---|---|---|---|---|
| Number of particles | $1\times10^7$ | $2.3\times10^5$ | $1.1\times10^4$ | $1.5\times10^3$ |
| Density of dispersion (number/ml) | $4\times10^7$ | $9\times10^5$ | $4.4\times10^5$ | $5.8\times10^4$ |
| *) Magnetic particles (DN) only in the case of particle size of 4.5 μm, and polystyrene particles (PS) in other cases. | | | | |

The change in the adhesion ratio of HT-1080 cells to each of the particles when the time of mixing each of the particles and the cancer cells was changed is shown in Fig. 5. The adhesion ratio of HT-1080 cells when the time of mixing each of the particles and the cancer cells was 15 minutes or 120 minutes is shown in Table 3.

Table 3

| Particle size (μm) | 4.5 (DN) | 20 (PS) | 90 (PS) | 200 to 300 (PS) |
|---|---|---|---|---|
| 15 minutes | 65% | 87% | 88% | 87% |
| 120 minutes | 74% | 90% | 92% | 87% |

It was confirmed in every case where HT-1080 cells had each particle size as shown in Fig. 5 and Table 3 that the HT-1080 cells could be adhered to particles at a high ratio and then separated from the solution by conducting mixing for at least 30 minutes. It was confirmed that in the case where particles having a particle size of approximately 10 μm or more were used, HT-1080 cells could be adhered at a sufficient ratio even by conducting mixing for a short time of approximately 15 minutes.

(2) Evaluation of cell adhesion morphology

The particles to which HT-1080 cells were adhered by the above-mentioned procedure were precipitated by centrifugation to separate the particles, and then washed with PBS, followed by inoculating the resultant in culture medium free from fetal bovine serum (FBS), and then observing the morphology of HT-1080 cells adhering to the particles by light microscopy.

The optical microscopic images of HT-1080 cells adhering to particles having a particle size of 4.5 μm or 20 μm are shown in Fig. 6. The morphology in which adhesion between the particles and the cell was formed by adhering plural particles around each cell was observed in the particles having a particle size of 4.5 μm. In contrast, it was observed in the particle having a particle size of 20 μm or more that the state in which each particle was monodispersed was maintained and the HT-1080 cells adhered to each monodispersed particle even after the particles and the cells were mixed. HT-1080 cells which did not adhere to the particles were observed in the culture medium, and the potential of the HT-1080 cells to self-sustainingly desorb from the particles after the particles were separated by centrifugation was suggested.

(3) Evaluation of self-sustaining desorption of cells from adhered particles

It was observed from the above-mentioned observation that the morphology of adhering HT-1080 cells varied depending on the particle size of the used cell-adhesive particles, and particularly that the HT-1080 cells self-sustainingly desorbed from the particles in the culture medium when the particle size was large. Accordingly, the phenomenon in which the HT-1080 cells separated from PBS by adhering to the particles subsequently desorbed from the particles was evaluated.

The PBS suspension in which the number of each of the particles coated with PMEA as mentioned above was adjusted to the number shown in Table 4 was mixed with the PBS suspension of HT-1080 cells as mentioned above, followed by fixing the mixture in a rotator to conduct agitation for 120 minutes to allow the HT-1080 cells to adhere to each of the particles. Then, the particles were precipitated and separated by centrifugation or applying a magnetic field thereto so as to prevent inclusion of cells which did not adhere to the particles, followed by adding PBS thereto to conduct washing, and then inoculating the resultant to the culture medium free from fetal bovine serum (FBS), cultivating in an incubator at 37°C in a 5% carbon dioxide atmosphere for 2 hours, and measuring the ratio of HT-1080 cells desorbed from the particles by using light microscopy. The measurement was conducted by counting visually the number of cells adhering to the particles and the number of cancer cells desorbed from the particles and moved into the culture medium within a range observed in a light microscope field of view (10-fold).

Table 4

| Particle size (μm) | 1.0 (DN) | 2.8 (DN) | 4.5 (DN) | 20 (PS) |
|---|---|---|---|---|
| Number of particles | $2\times10^8$ | $2.5\times10^7$ | $1\times10^7$ | $2.3\times10^5$ |
| Density of dispersion (number/ml) | $8\times10^8$ | $1\times10^8$ | $4\times10^7$ | $9\times10^5$ |
| *) Magnetic particles (DN) only in the case of particle size of 4.5 μm, and polystyrene particles (PS) in other cases. | | | | |

The desorption ratio of HT-1080 cells per particle size is shown in Fig. 7. The light micrographs of the particles and the cells observed above are shown in Fig. 8. It was observed as shown in Fig. 7 that the self-sustaining desorption ratio of cells adhering to particles varied depending on the particle size of the used particles, and when the particles having a particle size of 1.0 μm were used, the particles aggregated while adhering around the cells, and almost no HT-1080 cells were present alone in the culture medium (Fig. 8A). In contrast, it was observed that when the particles having a particle size of 4.5 μm were used, approximately 70% of HT-1080 cells self-sustainingly moved into the culture medium, and when the particles having a particle size of 20 μm were used, almost all cells self-sustainingly desorbed from the particles by maintaining the cells in the culture medium (Fig. 8B).

4. Adhesion test of human platelets to particles

The adhesion degree of platelets to each of the particles was confirmed so as to evaluate the possibility of separation from an aqueous solution by separating CTC or the like, which was present in blood, from blood cells such as platelets to selectively adhere the cells.

The human platelets to be used for evaluation were prepared as follows. Human whole blood was centrifuged (at 1,500 rpm for 5 minutes) to collect the supernatant, and a plasma component PRP containing a large amount of

platelets (platelet rich plasma) was obtained. In addition, another human whole blood was centrifuged (at 4,000 rpm for 10 minutes) to collect the supernatant, and a plasma component PPP containing a small amount of platelets (platelet poor plasma) was obtained. The PRP was diluted with the PPP to prepare a human platelet solution (platelet density: $1\times10^8$ platelets/ml) using a hemocytometer.

Magnetic particles (DN) and polystyrene particles (PS) coated or uncoated with PMEA, the number of which is shown in Table 5, were mixed with 0.5 ml of the above-mentioned human platelet solution (the number of platelets: $0.5\times10^8$ platelets) in tubes, respectively, fixed in a rotator, and then rotated at 3 rpm at room temperature for 60 minutes, followed by measuring the concentration of human platelets contained in the supernatant to calculate the adhesion ratio of human platelets to each of the particles.

Table 5

| | Coated with PMEA | Uncoated with PMEA |
|---|---|---|
| Magnetic particles Particle size: 4.5 μm, $2.0\times10^7$ particles | (0%) | 52% |
| Polystyrene particles Particle size: 20 μm, $4.5\times10^5$ particles | (0%) | 60% |

The above-evaluated adhesion ratios of human platelets to each of the particles are shown in Table 5. It was observed as shown in Table 5 that at least 50% of platelets adhered to particles regardless of the particle size of the particles by mixing with the particles uncoated with PMEA for 60 minutes. In contrast, the decrease in the number of platelets in the human platelet solution was not substantially observed after mixing with the particles coated with PMEA, which indicated that the adhesion frequency of platelets to particles was low.

In addition, each of the particles after the above-mentioned platelet adhesion test was washed with phosphate buffer, followed by adding 1% glutaraldehyde solution thereto to suspend the mixture, and then fixing the suspension in a rotator to rotate the resultant at room temperature at 3 rpm for 60 minutes to immobilize platelets adhered onto the particle surface, so as to confirm the adhesion of platelets onto the particle surface. Then, the particles were washed with phosphate buffer, pure water, and ethanol, and then placed on a PET substrate, followed by air-drying. The PET substrate was fixed onto a sample board dedicated to SEM (Scanning Electron Microscope) using a conductive tape, platinum-palladium was vacuum-deposited on the sample surface using an ion coater to obtain a SEM observation sample, and then observation was conducted using SEM (KEYENCE, VE-7800).

Results of the SEM observation of the particles after the above-mentioned platelet adhesion test are shown in Fig. 9. The presence of a significant adhering substance was confirmed on the surface of the particles uncoated with PMEA in comparison with the particles coated with PMEDA, as shown in Fig. 9, which indicated that the PMEA coating suppressed adhesion of platelets to the particle surface regardless of the quality of material of particles or the particle size.

The above-mentioned results indicate that in the case where magnetic particles or polystyrene particles, which are uncoated with PMEA, are mixed with the blood, platelets and the like, which are present in the blood at a high density, they preferentially adhere to the particle surface, and therefore it is substantially difficult for scarcely present cancer cells, stem cells, or the like, to adhere thereto.

In contrast, the substantial adhesion of platelets to particles having PMEA containing a predetermined intermediate water content when hydrated on the surface thereof was not observed, whilst a high adhesion ratio of tumor cells or the like was observed, which indicated that selective cell adhesion was caused. The results are identical to evaluation results of a flat membrane sample reported by the present inventors in Patent Document 3, and indicate that the presence of intermediate water on the particle surface allows predetermined cells to selectively adhere depending on the intermediate water content. In addition, it is suggested that the use of particles containing intermediate water on the surface thereof in an amount corresponding to the adhesion target when hydrated makes it possible to allow cancer cells, stem cells, or the like to selectively adhere thereto in the blood in which blood cells are present at a high density.

5. Adhesion test of cancer cells in mimic blood

The use of particles having on the surface thereof a wettable composition exhibiting a predetermined amount of intermediate water by hydration allowed tumor cells or the like to selectively adhere thereto, as shown above, and in the case where the particle size thereof was approximately 3 μm or more, the adhering cells self-sustainingly desorbed to move to the surface of a culture dish or the like, which indicated that subsequent cultivation can be conducted favorably.

Therefore, the cell-adhesive particles according to the present invention were added to an atmosphere in which both tumor cells and platelets were present together to selectively adhere tumor cells to separate the tumor cells from platelets, followed by collecting the tumor cells in the culture medium.

The dispersion medium of the PBS suspension of the HT-1080 cells (cell concentration in the suspension: $2.0\times10^6$ cells/ml) was replaced with the plasma component PPP, and then the plasma component PRP containing a

large amount of platelets was mixed therewith to prepare a suspension in which the HT-1080 cells (cell concentration: $2.0\times10^6$ cells/ml) and human platelets (platelet concentration: $1.0\times10^8$ platelets/ml) were suspended. 0.25 ml of the resultant suspension was mixed with a PBS suspension in which $2.3\times10^5$ polystyrene particles coated with PMEA (particle size: 20 μm) were suspended at a concentration of $9.0\times10^5$ particles/ml in a tube, and then the resultant suspension was fixed in a rotator, and rotated at room temperature at 3 rpm for 60 minutes, followed by measuring the concentration of the HT-1080 cells and human platelets contained in the resultant supernatant to calculate each adhesion ratio to the particles.

In addition, the mixture prepared as mentioned above was rotated for 120 minutes, and then subjected to centrifugation to precipitate and separate the cell-adhesive particles, followed by removing the supernatant from the resultant, conducting washing with PBS, inoculating the resultant in culture medium free from fetal bovine serum (FBS), conducting cultivation in an incubator at 37°C in a 5% carbon dioxide atmosphere for 24 hours, and then observing cell appearance under a light microscope, so as to confirm the state of the HT-1080 cells adhered and then separated by the cell-adhesive particles.

Adhesion ratios of HT-1080 cells and human platelets measured above are shown in Table 6. In addition, a light micrograph of the HT-1080 cells observed above is shown in Fig. 10. It was confirmed as shown in Table 6 that the particles coated with PMEA, the particle size of which was 20 μm, captured the HT-1080 cells at a high ratio while suppressing adhesion of the platelets, which suggested that selective adhesion of tumor cells can be realized by the cell-adhesive particles according to the present invention.

Table 6

| Cell type | HT-1080 cells | Human platelets |
|---|---|---|
| Adhesion ratio | 96% | 2% |

Since it was observed as shown in Fig. 10 that the HT-1080 cells adhering to the particles desorbed self-sustainingly from the particles, adhered to a culture dish, and then elongated therein by inoculating the separated particles into culture medium to conduct cultivation, it is possible to use the cells captured by the cell-adhesive particles according to the present invention to conduct various evaluations.

## Claims

1. A cell-adhesive particle adherable to highly adherent cells present in an aqueous solution, comprising a wettable composition having an intermediate water content of 1% by weight to 30% by weight when saturatedly hydrated on at least a part of a surface.

2. The cell-adhesive particle according to claim 1, wherein an average particle size is 2 μm to 500 μm.

3. The cell-adhesive particle according to claim 1 or 2, wherein the highly adherent cells comprise tumor cells, stem cells, vascular endothelial cells, nerve cells, macrophages, dendritic cells, monocytes, neutrophils, smooth muscle cells, fibroblasts, cardiac muscle cells, skeletal muscle cells, hepatic parenchymal cells, hepatic nonparenchymal cells, or pancreatic islet cells.

4. The cell-adhesive particle according to any one of claims 1 to 3, wherein the wettable composition is a polymer comprising methoxyethyl acrylate.

5. A cell capture method comprising adhering highly adherent cells by contacting a particle comprising a wettable composition having an intermediate water content of 1% by weight to 30% by weight when saturatedly hydrated on at least a part of a surface with a solution comprising the highly adherent cells.

6. The cell capture method according to claim 5, wherein an average particle size of the cell-adhesive particle is 2 μm to 500 μm.

7. The cell capture method according to claim 5 or 6, wherein the highly adherent cells comprise tumor cells, stem cells, vascular endothelial cells, nerve cells, macrophage, dendritic cells, monocytes, neutrophils, smooth muscle cells, fibroblasts, cardiac muscle cells, skeletal muscle cells, hepatic parenchymal cells, hepatic nonparenchymal cells, or pancreatic islet cells.

**8.** The cell capture method according to any one of claims 5 to 7, wherein the wettable composition is a polymer comprising methoxyethyl acrylate.

**9.** A particle-cell complex, comprising: a particle comprising a wettable composition having an intermediate water content of 1% by weight to 30% by weight when saturatedly hydrated on at least a part of a surface; and highly adherent cells adhered on the surface of the particle.

**10.** The particle-cell complex according to claim 9, wherein an average particle size of the particle is 2 μm to 500 μm.

**11.** The particle-cell complex according to claim 9 or 10, wherein the highly adherent cells comprise tumor cells, stem cells, vascular endothelial cells, nerve cells, macrophages, dendritic cells, monocytes, neutrophils, smooth muscle cells, fibroblasts, cardiac muscle cells, skeletal muscle cells, hepatic parenchymal cells, hepatic nonparenchymal cells, or pancreatic islet cells.

**12.** The particle-cell complex according to any one of claims 9 to 11, wherein the wettable composition is a polymer comprising methoxyethyl acrylate.

**13.** A method for desorbing cells from a particle-cell complex of any one of claims 10 to 12, comprising inoculating the particle-cell complex in a cell culture medium.

**14.** A cell-cultivation method comprising cultivating cells obtained by a method of claim 13 in a cell culture medium.

*FIG. 1*

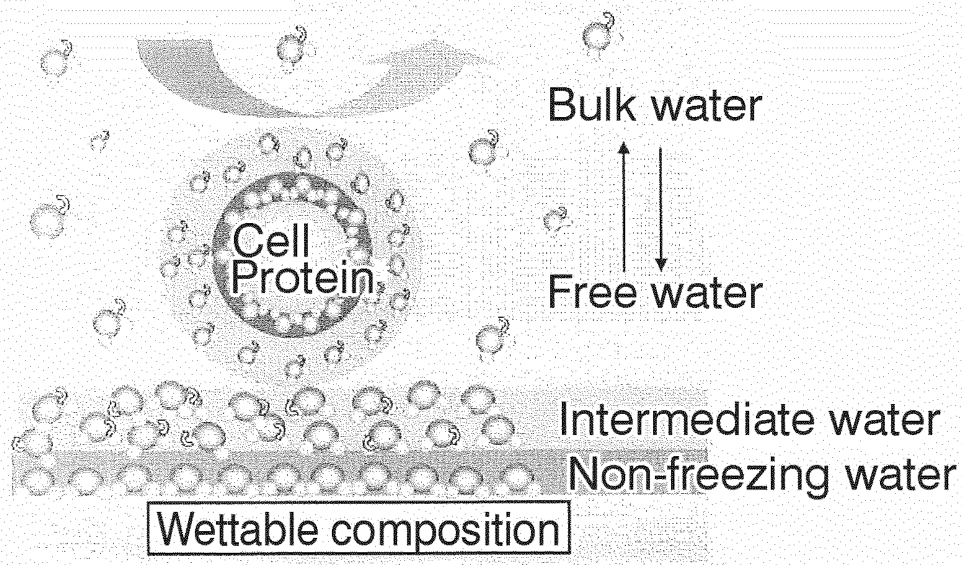
Bulk water
Free water
Cell
Protein
Intermediate water
Non-freezing water
Wettable composition

*FIG. 2*

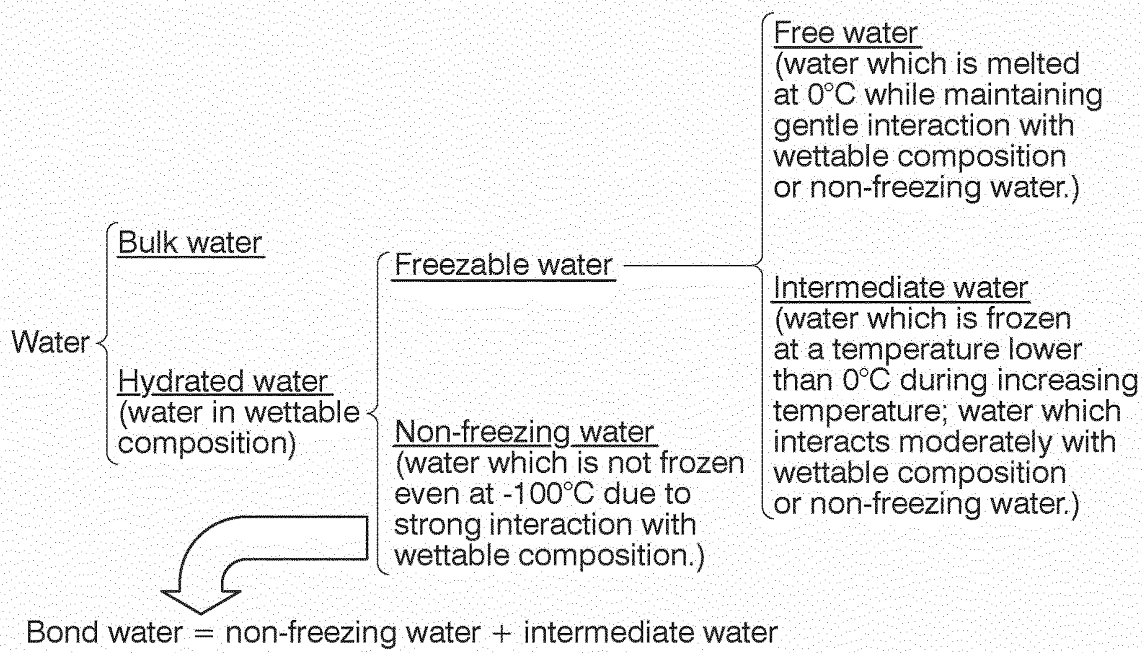
Water
Bulk water
Hydrated water
(water in wettable composition)
Freezable water
Non-freezing water
(water which is not frozen even at -100°C due to strong interaction with wettable composition.)
Free water
(water which is melted at 0°C while maintaining gentle interaction with wettable composition or non-freezing water.)
Intermediate water
(water which is frozen at a temperature lower than 0°C during increasing temperature; water which interacts moderately with wettable composition or non-freezing water.)
Bond water = non-freezing water + intermediate water

FIG. 3A

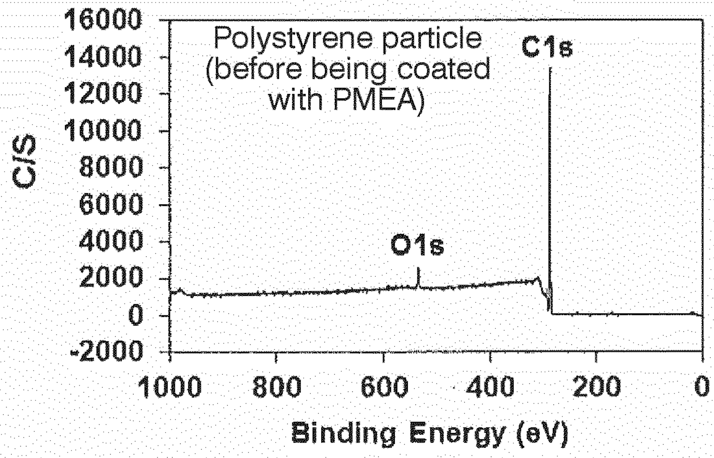


FIG. 3B

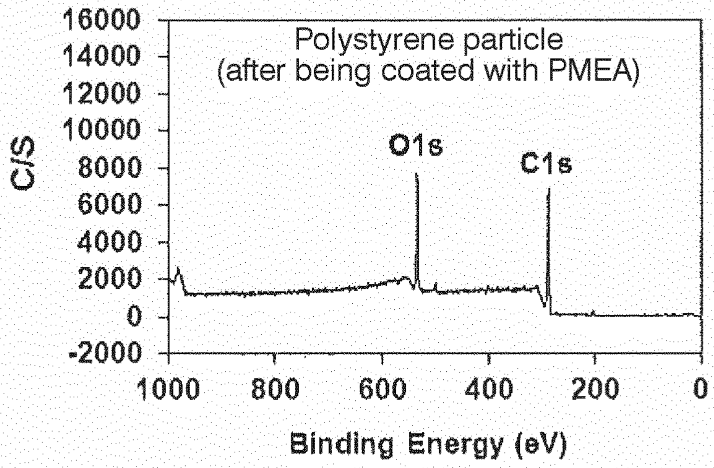


FIG. 4A

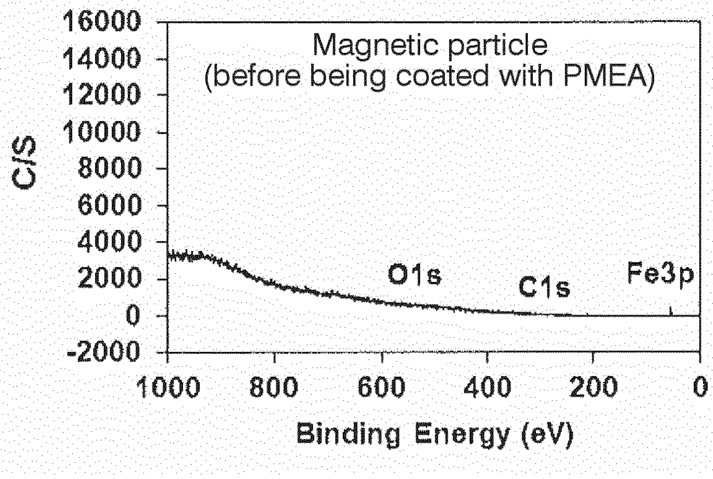

*FIG. 4B*

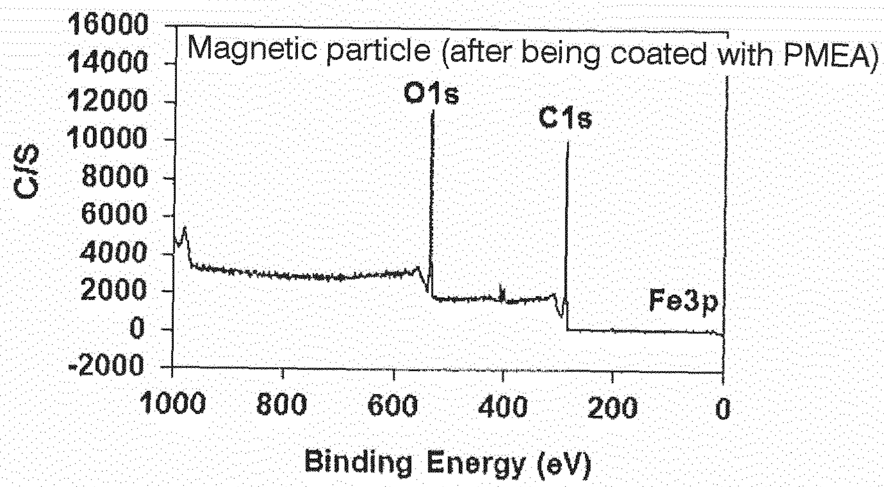
Magnetic particle (after being coated with PMEA)
O1s
C1s
Fe3p
C/S
16000
14000
12000
10000
8000
6000
4000
2000
0
-2000
1000
800
600
400
200
0
Binding Energy (eV)

*FIG. 5*

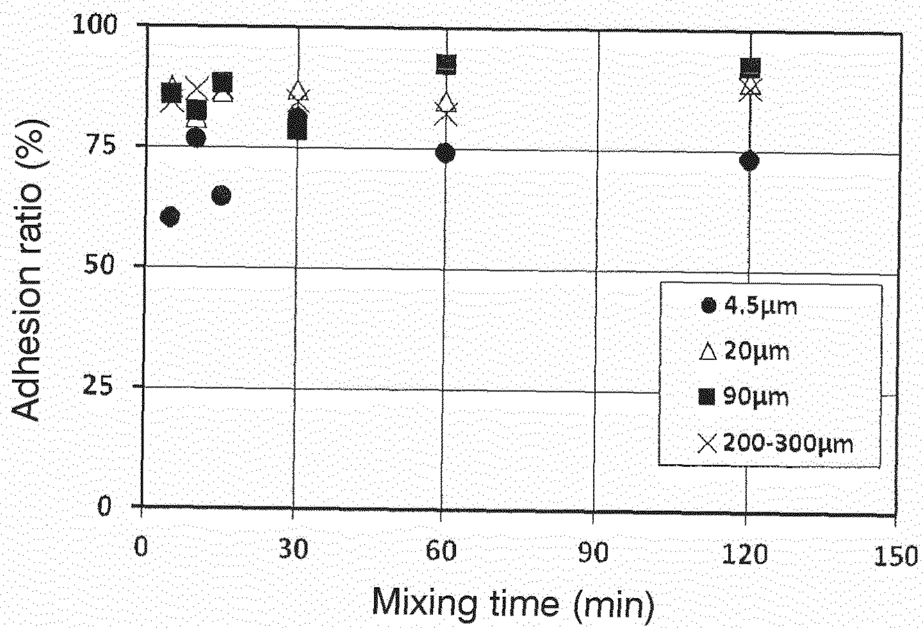
Adhesion ratio (%)
100
75
50
25
0
0
30
60
90
120
150
Mixing time (min)
4.5µm
20µm
90µm
200-300µm

FIG. 6A

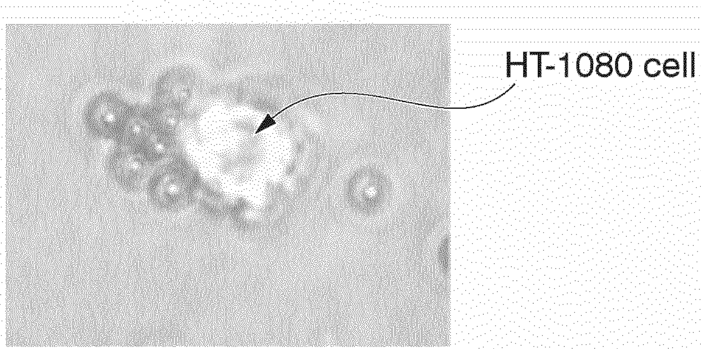


Magnetic particles coated with PMEA (4.5 $\mu$m)

FIG. 6B

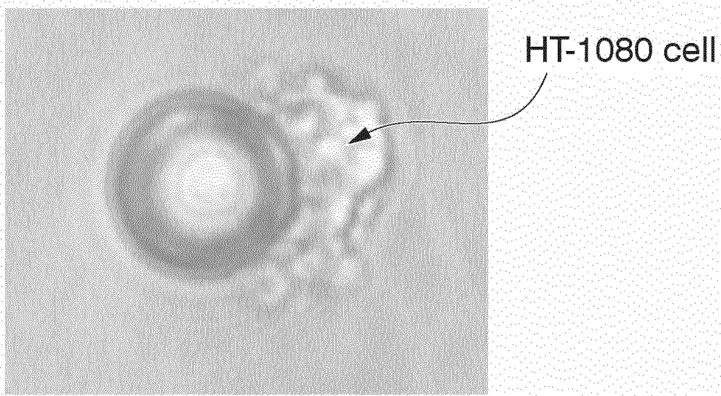


Magnetic particle coated with PMEA (20 $\mu$m)

FIG. 7

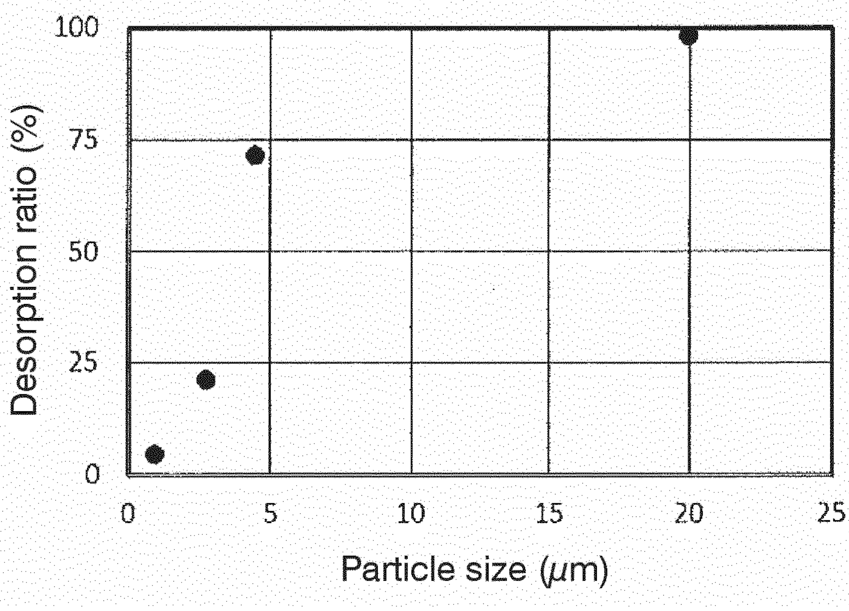

FIG. 8A

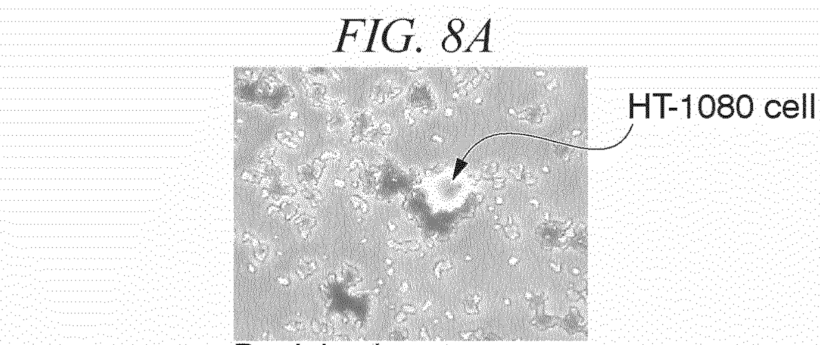


Particle size: 1 μm

FIG. 8B

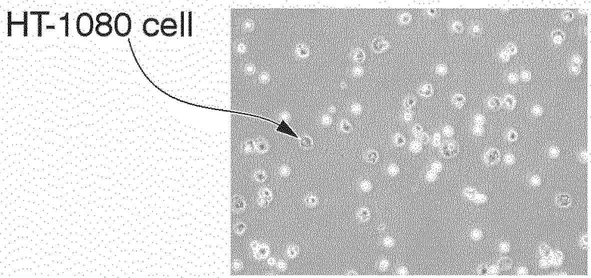


Particle size: 20 μm

FIG. 9A

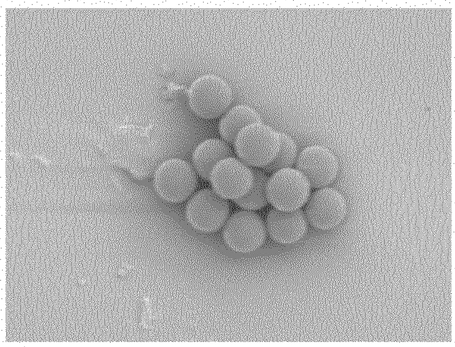

Magnetic particles (4.5 μm) coated with PMEA

FIG. 9B

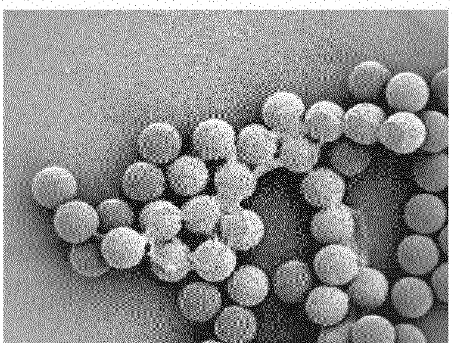

Magnetic particles (4.5 μm) uncoated with PMEA

FIG. 9C

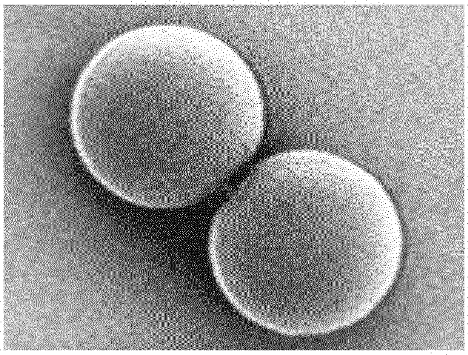

Polystyrene particles (20 μm) coated with PMEA

FIG. 9D

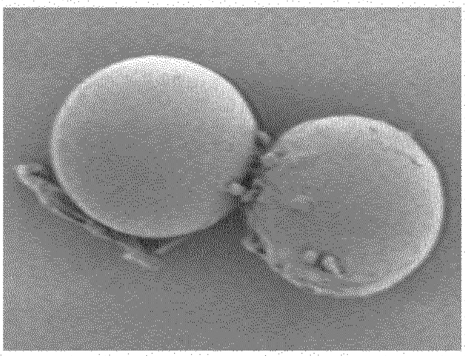

Polystyrene particles (20 μm) uncoated with PMEA

FIG. 10

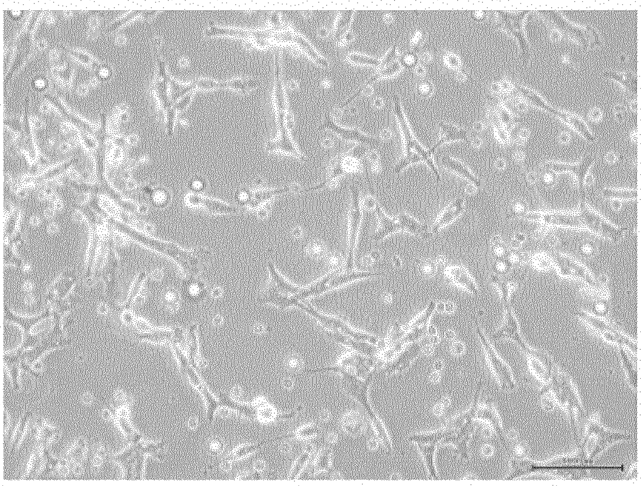

## INTERNATIONAL SEARCH REPORT

International application No. PCT/JP2020/014538

**A. CLASSIFICATION OF SUBJECT MATTER**

Int.Cl. C08F20/28(2006.01)i, C12M1/26(2006.01)i, C12M3/00(2006.01)i, C12N5/07(2010.01)i, C12N5/071(2010.01)i, C12N5/0735(2010.01)i, C12N5/074(2010.01)i, C12N5/077(2010.01)i, C12N5/0775(2010.01)i, C12N5/0784(2010.01)i, C12N5/0786(2010.01)i, C12N5/0787(2010.01)i, C12N5/0789(2010.01)i, C12N5/0793(2010.01)i, C12N5/09(2010.01)i, C12N1/00(2006.01)i, C12N1/02(2006.01)i, G01N33/48(2006.01)i, C12N11/087(2020.01)i

FI: C12M3/00A, C12N11/087, C12M1/26, C12N1/02, C12N5/07, C12N5/0735, C12N5/074, C12N5/0775, C12N5/0786, G01N33/48S, C12N5/0787, C12N5/0789, C12N5/077, C12N5/071, C12N5/0793, C12N5/09, C12N1/00K, C08F20/28, C12N5/0784

According to International Patent Classification (IPC) or to both national classification and IPC

**B. FIELDS SEARCHED**

Minimum documentation searched (classification system followed by classification symbols)

Int.Cl. C12M1/00-3/10, C08F6/00-246/00, C08F301/00, C12N1/00-15/90, G01N33/00-33/98

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

JSTPlus/JMEDPlus/JST7580(JDreamIII), CAplus/MEDLINE/EMBASE/BIOSIS/WPIDS(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2016-063801 A (YAMAGATA UNIV.) 28.04.2016 (2016-04-28), claims, paragraphs [0017], [0035], [0037], examples | 1, 3-5, 7-9, 11-14<br>2, 6, 10 |
| X<br>Y | JP 2012-105579 A (YAMAGATA UNIV.) 07.06.2012 (2012-06-07), claims, paragraphs [0010], [0023], [0032], examples | 1, 3-5, 7-9, 11-14<br>2, 6, 10 |
| Y | JP 2005-525407 A (INSERM) 25.08.2005 (2005-08-25), claims | 2, 6, 10 |

☒ Further documents are listed in the continuation of Box C.

☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| | |
|---|---|
| Date of the actual completion of the international search<br>23.04.2020 | Date of mailing of the international search report<br>16.06.2020 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2020/014538

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2018-042572 A (DAI NIPPON PRINTING CO., LTD.) 22.03.2018 (2018-03-22), claims | 2, 6, 10 |
| Y | JP 2003-189848 A (SANYO CHEMICAL IND LTD.) 08.07.2003 (2003-07-08), claims | 2, 6, 10 |

Form PCT/ISA/210 (second sheet) (January 2015)

**INTERNATIONAL SEARCH REPORT**
Information on patent family members

International application No.
PCT/JP2020/014538

| | | |
|---|---|---|
| JP 2016-063801 A | 28.04.2016 | (Family: none) |
| JP 2012-105579 A | 07.06.2012 | (Family: none) |
| JP 2005-525407 A | 25.08.2005 | US 2005/0129776 A1 claims<br>EP 1507517 A1 |
| JP 2018-042572 A | 22.03.2018 | (Family: none) |
| JP 2003-189848 A | 08.07.2003 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2019069462 A
- JP 2019079696 A
- JP 2002503814 W
- JP 2010104350 A
- JP 2012105579 A
- JP 2016063801 A
- JP 2016131561 A

### Non-patent literature cited in the description

- *Biomaterial,* 2010, vol. 28-1, 34-46